# EUROPÄISCHE PATENTSCHRIFT

(11) **EP 1 850 135 B1**
(45) Veröffentlichungstag und Bekanntmachung des Hinweises auf die Patenterteilung: **03.11.2010**
(21) Anmeldenummer: 07007504.9
(22) Anmeldetag: 12.04.2007
(51) Int. Cl.: G01N 33/86

(54) **Verfahren zur Bestimmung der Thrombozytenfunktion und der Flussbedingungen**
Method for determining the thrombocyte function and flow conditions
Procédé de détermination de la fonction des thrombocytes et des conditions liquides

(30) Priorität: 28.04.2006 DE 102006020386
(43) Veröffentlichungstag der Anmeldung: 31.10.2007
(73) Patentinhaber: Siemens Healthcare Diagnostics Products GmbH, 35041 Marburg (DE)
(72) Erfinder: Rechner, Andreas, Dr., 35043 Marburg (DE)

(56) Entgegenhaltungen:
- EP-A1- 0 716 744
- WO-A-02/074322
- WO-A-2005/007868
- MISCHKE R ET AL: "Influence of platelet count, acetylsalicylic acid, von Willebrand's Disease, coagulopathies, and haematocrit on results obtained using a platelet function analyser in dogs" VETERINARY JOURNAL, BAILLIERE TINDALL, LONDON,, GB, Bd. 165, Nr. 1, Januar 2003 (2003-01), Seiten 43-52, XP002437870 ISSN: 1090-0233
- BIN XU ET AL: "Acyclic Analogues of Adenosine Biphosphates as P2Y Receptor Antagonists: Phosphate Substitution Leads to Multiple Pathways of Inhibition of Platelet Aggregation" JOURNAL OF MEDICINAL CHEMISTRY, AMERICAN CHEMICAL SOCIETY. WASHINGTON, US, Bd. 45, 2002, Seiten 5694-5709, XP002285317 ISSN: 0022-2623
- "PROD. NO. M5942 - MRS2395 CELL SIGNALING AND NEUROSCIENCE FIRST P2Y12 RECEPTOR ANTAGONIST AVAILABLE FROM SIGMA-RBI" SIGMA ALDRICH CATALOG, XX, XX, 2002, Seite 1, XP002437879
- JUDGE H M ET AL: "Incomplete P2Y12 receptor blockade by Clopidogrel is not due to the presence of an internal pool of P2Y12 Receptors" JOURNAL OF THROMBOSIS AND HAEMOSTASIS, BLACKWELL PUBLISHING, OXFORD, GB, Bd. 3, Nr. Supplement1, August 2005 (2005-08), Seite P2148, XP002437871 ISSN: 1538-7933
- WIVIOTT STEPHEN D ET AL: "Randomized comparison of prasugrel (CS-747, LY640315), a novel thienopyridine P2Y12 antagonist, with clopidogrel in percutaneous coronary intervention: results of the Joint Utilization of Medications to Block Platelets Optimally (JUMBO)-TIMI 26 trial" CIRCULATION, LIPPINCOT WILLIAMS AND WILKINS, BALTIMORE, US, Bd. 111, Nr. 25, 28. Juni 2005 (2005-06-28), Seiten 3366-3373, XP002437872 ISSN: 1524-4539
- KAM P C A ET AL: "The thienopyridine derivatives (platelet adenosine diphosphate receptor antagonists), pharmacology and clinical developments" ANAESTHESIA, ACADEMIC PRESS, LONDON, GB, Bd. 58, Nr. 1, Januar 2003 (2003-01), Seiten 28-35, XP002437873 ISSN: 0003-2409
- KOZEK-LANGENECKER S A ET AL: "[Locoregional anesthesia and coagulation inhibitors. Recommendations of the Task Force on Perioperative Coagulation of the Austrian Society for Anesthesiology and Intensive Care Medicine] LOKOREGIONALAESTHESIEN UNTER GERINNUNGSHEMMENDER MEDIKATION. EMPFEHLUNGEN DER ARBEITSGRUPPE PERIOPERATIVE GERINN" ANAESTHESIST, SPRINGER VERLAG, DE, Bd. 54, Nr. 5, Mai 2005 (2005-05), Seiten 476-484, XP002437874 ISSN: 0003-2417
- ANDRE PATRICK ET AL: "Anticoagulants (thrombin inhibitors) and aspirin synergize with P2Y12 receptor antagonism in thrombosis" CIRCULATION, LIPPINCOT WILLIAMS AND WILKINS, BALTIMORE, US, Bd. 108, Nr. 21, 25. November 2003 (2003-11-25), Seiten 2697-2703, XP002437875 ISSN: 1524-4539
- HOUSTON DAYLE ET AL: "[P-32]2-iodo-N-6-methyl-(N)-methanocarba- 2 '-deoxyadenosine-3 ',5 '-bisphosphate ([P-32]MRS2500), a novel radioligand for quantification of native P2Y(1) receptors" BRITISH JOURNAL OF PHARMACOLOGY, BASINGSTOKE, HANTS, GB, Bd. 147, Nr. 5, März 2006 (2006-03), Seiten 459-467, XP002437876 ISSN: 0007-1188
- JIN JIANGUO ET AL: "Adenosine diphosphate (ADP)-induced thromboxane A2 generation in human platelets requires coordinated signaling through integrin alphaIIbbeta3 and ADP receptors" BLOOD, W.B.SAUNDERS COMPANY, ORLANDO, FL, US, Bd. 99, Nr. 1, 1. Januar 2002 (2002-01-01), Seiten 193-198, XP002437877 ISSN: 0006-4971
- MIYAMOTO K ET AL: "Stimulatory and inhibitory effects of forskolin on adenylate cyclase in rat normal hepatocytes and hepatoma cells." JOURNAL OF PHARMACOBIO-DYNAMICS FEB 1989, Bd. 12, Nr. 2, Februar 1989 (1989-02), Seiten 87-93, XP002441664 ISSN: 0386-846X

## Beschreibung

Die Erfindung liegt auf dem Gebiet der Thrombyzytenfunktionsdiagnostik und betrifft ein in vitro-Verfahren zur Bestimmung der Thrombozytenfunktion unter Flussbedingungen. Das Verfahren eignet sich insbesondere zur Bestimmung der Wirkung von Clopidogrel nach oraler Einnahme und anderer antithrombotisch wirksamer P2Y(12)-Antagonisten sowie zur Bestimmung antithrombotisch wirksamer Antogonisten des P2Y(1)-Rezeptors.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z. B. die Thrombozyten (Plättchen). Im Falle einer Gefäßverletzung kommt es zuerst zur Anlagerung von Thrombozyten an das subendotheliale Kollagen. Diese Adhäsion wird durch Adhäsivproteine, wie den von-Willebrand-Faktor (VWF) vermittelt. Während des Adhäsionsvorgangs werden die Thrombozyten aktiviert und schütten Mediatoren aus ihren Granulae aus, wodurch die Aggregation weiterer Thrombozyten sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

In der Gerinnungsdiagnostik wurden verschiedene in vitro-Testverfahren entwickelt, mit deren Hilfe bestimmt werden kann, ob das Blut eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Eine wichtige Teilfunktion des Gerinnungssystems, die gezielt untersucht werden kann, ist die primäre Hämostase, die wesentlich von der Funktionsfähigkeit der Thrombozyten abhängt.

Verfahren zur Bestimmung der Thrombozytenfunktion werden nicht nur zur Diagnose von erworbenen oder angeborenen Thrombozytenfunktionsstörungen verwendet, sondern auch zur Überwachung antithrombotischer Therapien. Medikamente, die die Aggregation von Thrombozyten hemmen, werden vorwiegend zur Prophylaxe und Therapie arterieller thromboembolischer Ereignisse, wie Myokardinfarkt oder Schlaganfall eingesetzt. Die verbreitetsten Wirkstoffe mit thrombozytenaggregationshemmender Wirkung sind Acetylsalicylsäure (ASS) und die Thienopyridine Clopidogrel und Ticlopidin. ASS hemmt irreversibel Cyclooxygenase-1 (CQX-1), ein intrazelluläres Enzym, das an der Synthese von dem thrombozytenaggregationsfördernden Thromboxan A2 beteiligt ist. Clopidogrel und Ticlopidin gehören aufgrund ihres Wirkungsmechanismus zur Klasse der P2Y(12)-Antagonisten. Nach oraler Einnahme von Clopidogrel oder Ticlopidin entstehen in der Leber Metabolite, die selektiv den P2Y(12)-Rezeptor blockieren. Der purinerge P2Y(12)-Rezeptor wird auf der Thrombozytenoberfläche exprimiert und ist durch extrazelluläres Adenosin-5'-diphosphat (ADP) aktivierbar. Infolge der Aktivierung des P2Y(12)-Rezeptors werden in den Thrombozyten intrazelluläre Prozesse, wie zum Beispiel die Inhibierung der Bildung von cAMP, in Gang gesetzt, die eine Thrombozytenaggregationsreaktion hervorrufen. P2Y(12)-Antagonisten blockieren die P2Y(12)-Rezeptoren auf der Thrombozytenoberfläche und wirken daher antithrombotisch.

Der zweite purinerge ADP-Rezeptor P2Y(1) wird ebenfalls auf der Thrombozytenoberfläche exprimiert und durch extrazelluläres Adenosin-5'-diphosphat aktiviert. Infolge der Aktivierung des P2Y(1)-Rezeptors werden in den Thrombozyten intrazelluläre Prozesse, wie zum Beispiel der Anstieg des intrazellulären Calciums, in Gang gesetzt, die eine Thrombozytenaggregationsreaktion hervorrufen. Antagonisten des P2Y(1)-Rezeptors wirken diesem Prozess entgegen und wirken daher antithrombotisch.

Ein genaues Wissen um den Status der Thrombozytenfunktion von Patienten, die eine antithrombotische Therapie erhalten, wird als immer wichtiger erachtet, da z. B. das Auftreten einer sogenannten Clopidogrelresistenz als ernst zu nehmender Risikofaktor betrachtet wird. Eine Clopidogrelresistenz liegt vor, wenn die Thrombozytenfunktion eines Patienten durch die Gabe der Standarddosis Clopidogrel nicht oder kaum beeinflusst wird. Durch die Bestimmung der Thrombozytenfunktion kann einerseits überprüft werden, ob durch die gewählte Dosierung tatsächlich eine ausreichende antithrombotische Wirkung erzielt wird. Andererseits können zu hohe Dosierungen oder Wirkungen eines antithrombotischen Medikaments festgestellt und behandelt werden, was z. B. vor operativen Eingriffen zum Ausschluss von Blutungskomplikationen notwendig ist.

Im Stand der Technik sind verschiedene Verfahren zur Untersuchung der Thrombozytenfunktion bekannt. Bei der Blutungszeitbestimmung handelt es sich um einen in vivo-Globaltest, der die primäre Hämostase erfasst. Die Blutungszeit wird bestimmt, indem dem Patienten eine kleine Schnitt- oder Stichverletzung zugefügt wird und die Zeit bis zum Blutungsstillstand gemessen wird. Es handelt sich um einen schwer standardisierbaren, grob informativen Test, der vor allem in Notfallsituationen angewendet wird, um einen Überblick über die primäre Hämostase zu erhalten. Die Einnahme von Thrombozytenaggregationshemmem führt zu einer Verlängerung der Blutungszeit. Nachteilig an der Blutungszeitbestimmung ist, dass bei einer normalen Blutungszeit eine Thrombozytenfunktionsstörung nicht ausgeschlossen werden kann.

Verschiedene in vitro-Verfahren ermöglichen einen wesentlich empfindlicheren Nachweis von Thrombozytenfunktionsstörungen. Bei diesen Verfahren wird üblicherweise in einer Vollblutprobe oder in einer Probe von plättchenreichem Plasma (PRP) die Aggregation der Thrombozyten durch Zugabe eines Aktivators induziert und die Aggregationsreaktion gemessen. Die meistverwendeten Aktivatoren, die zur Induktion der Thrombozytenaggregation verwendet werden, sind: ADP (Adenosin-5'-diphosphat), Kollagen, Epinephrin (Adrenalin), Ristocetin und verschiedene Kombinationen davon sowie Thrombin, TRAP (Thrombin-Receptor-Activating-Protein) oder Serotonin.

Bei der Lichttransmissionsaggregometrie, die auch als Plättchenaggregation nach Born bezeichnet wird, wird die Aggregationsfähigkeit der Thrombozyten im plättchenreichen Plasma in Gegenwart von aggregationsinduzierenden Substanzen in einem Aggregometer photometrisch gemessen. Durch die Aggregatbildung wird die Lichtdurchlässigkeit der PRP-Probe erhöht, so dass durch die Messung der Lichtdurchlässigkeit z. B. die Geschwindigkeit der Aggregatbildung bestimmt werden kann. Mit Hilfe der Lichttransmissionsaggregometrie können auch therapeutische Effekte von medikamentös eingesetzten Thrombozytenaggregationshemmem erfasst werden. Ein Nachteil der Lichttransmissionsaggregometrie ist, dass ausschließlich plättchenreiches Plasma als Probenmaterial verwendet werden kann. Plättchenreichem Plasma fehlen nicht nur wichtige Bestandteile des Blutes, wie z. B. die roten und weißen Blutkörperchen, sondern es erfordert außerdem eine zeitaufwändige und fehleranfällige Probenvorbereitung.

Ein anderes Testprinzip zur Bestimmung der Thrombozytenfunktion ist im Platelet Function Analyzer (PFA-100^{®}, Dade Behring Marburg GmbH, Marburg, Deutschland) verwirklicht. Der PFA-100^{®} ist ein globaler, automatisierter und standardisierter in vitro-Vollbluttest, bei dem die primäre Hämostase unter Flussbedingungen und damit in Gegenwart von hohen Scherkräften gemessen wird. Zur Simulation der Flussbedingungen und der Scherkräfte, wie sie in kleineren arteriellen Blutgefäßen herrschen, wird in einer speziellen Messzelle ein Unterdruck von etwa -40 mbar erzeugt und das Citratvollblut, dass sich in einem Probenreservoir befindet, strömt durch eine Kapillare, die einen Durchmesser von etwa 200 µm hat. Die Kapillare mündet in eine Messkammer die mit einem Trennelement, z. B. einer Membran abgeschlossen ist, welche eine kapillare zentrale Öffnung (Apertur) enthält, durch die das Blut aufgrund des Unterdrucks durchtritt. In den meisten Fällen ist die Membran, zumindest in dem Bereich um die Apertur mit einem oder mehreren Aktivatoren, die die Thrombozytenaggregation induzieren, versetzt, so dass das vorbeiströmende Blut mit den aggregationsinduzierenden Substanzen im Bereich der Apertur in Kontakt kommt. Infolge der induzierten Adhäsion und Aggregation der Thrombozyten bildet sich im Bereich der Apertur ein Thrombozytenpfropf (Thrombus), der die Membranöffnung verschließt und den Blutfluss stoppt. In diesem System wird die Zeit gemessen, die bis zum Verschluss der Membranöffnung benötigt wird. Diese sogenannte Verschlusszeit korreliert mit der Funktionsfähigkeit der Thrombozyten. Eine Messzelle zur Verwendung in einem Verfahren zu Bestimmung der Thrombozytenfunktion anhand der Verschlusszeit ist z. B. in dem Patentdokument WO 97/34698 beschrieben. Bislang werden in dem Verfahren zur Bestimmung der Verschlusszeit Messzellen verwendet, die mit einer Membran ausgestattet sind, die mit Kollagen (Kol) und zusätzlich entweder mit ADP oder mit Epinephrin (Epi) beschichtet ist. Je nach Ausstattung werden also Kol/ADP-Messzellen und Kol/Epi-Messzellen unterschieden. Üblicherweise wird eine Patientenprobe zunächst mit Hilfe einer Kol/Epi-Messzelle analysiert. Im Falle einer abnormal verlängerten Kol/Epi-Verschlusszeit, die auf eine Störung der Thrombozytenaggregation hinweist, wird im Anschluss eine Kol/ADP-Messung durchgeführt. Ist die Kol/ADP-Verschlusszeit ebenfalls abnormal verlängert, ist dies ein Indikator für eine gestörte Thrombozytenfunktion oder für eine Störung des von-Willebrand-Faktors. Ist die Kol/ADP-Verschlusszeit hingegen normal, so kann dies auf die Gegenwart von Acetylsalicylsäure hinweisen oder auf das Vorliegen einer erworbenen oder angeborenen Thrombozytopathie, wie z. B. der Storage Pool Disease. Ein Nachteil des PFA-100^{®} Systems ist, dass die zur Verfügung stehenden Kol/ADP- und Kol/Epi-Messzellen nur eine begrenzte Empfindlichkeit für den aggregationshemmenden Effekt von Thrombozytenaggregationshemmern aus der Gruppe der Thienopyridine (z. B. Clopidogrel, Ticlopidin) aufweisen. Eine zuverlässige Bestimmung des therapeutischen Effekts von medikamentös eingesetztem Clopidogrel oder Ticlopidin, insbesondere wenn der Patient zusätzlich ASS (z. B. Aspirin^{®}) eingenommen hat, ist mit Hilfe der bekannten Kol/ADP- und Kol/Epi-Messzellen im PFA-100^{®}-System bislang nicht möglich.

Das Patentdokument WO 2005/007868 A2 beschreibt ein anderes Verfahren zur Bestimmung der Thrombozytenfunktion, welches den Nachweis der therapeutischen Wirkung von Clopigogrel und anderen P2Y(12)-Antagonisten ermöglicht. Bei diesem Verfahren wird eine Vollblutprobe eines Patienten mit einem Antikoagulanz gemischt und mit ADP zur Induktion der Thrombozytenaggregation versetzt. Außerdem wird der Probe Prostaglandin E1 (PGE 1) zugegeben. Prostaglandin E1, ein Produkt des menschlichen Arachidonsäuremetabolismus, vermag bereits in geringen Dosen die Reaktivität von Thrombozyten deutlich zu reduzieren und wird daher auch zur Hemmung der Thrombozytenaktivierung eingesetzt. In dem in WO 2005/007868 A2 beschriebenen Testverfahren wird PGE 1 eingesetzt, um die unerwünschte Aktivierung des ADP-Rezeptors P2Y(1) zu verhindern und damit die Spezifität des Testverfahrens für den P2Y(12)-Rezeptor bzw. für P2Y(12)-Antagonisten wie Clopidogrel zu erhöhen. Zusätzlich werden Mikropartikel zugegeben, an die ein Ligand des GPllb/llla-Rezeptors, wie z. B. Fibrinogen gekoppelt ist, und die Aggregationsreaktion wird anhand der zunehmenden Lichtdurchlässigkeit aggregometrisch gemessen. Nachteilig an dem vorbeschriebenen Verfahren ist, dass die Thrombozytenfunktion wie bei der Lichttransmissionsaggregometrie nicht unter dem Einfluss von Flussbedingungen und Scherkräften bestimmt wird.

Der vorliegenden Erfindung lag die Aufgabe zugrunde, ein sensitives Verfahren zur Bestimmung der Thrombozytenfunktion unter Flussbedingungen bereitzustellen, das es insbesondere ermöglicht, die antithrombotische Wirkung von P2Y(12)-Antagonisten zu bestimmen. Die Lösung der Aufgabe besteht in der Bereitstellung der in den Ansprüchen beschriebenen erfindungsgemäßen Verfahren.

Gegenstand der vorliegenden Erfindung ist ein in vitro-Verfahren zur Bestimmung der Thrombozytenfunktion in einer Vollblutprobe. Bevorzugterweise handelt es sich bei der Vollblutprobe um frisch abgenommenes, venöses, antikoaguliertes menschliches oder tierisches Blut, das innerhalb von vier Stunden nach der Blutentnahme mit Hilfe des erfindungsgemäßen Verfahrens untersucht werden sollte. Bevorzugterweise wird das Vollblut durch Zugabe eines Antikoagulanzes antikoaguliert. Zur Verwendung als Antikoagulanz geeignet sind gepufferte, Calcium-bindende Citratlösungen, wie z. B. 3,2 oder 3,8 %ige gepufferte Natrimcitratlösungen, sowie natürliche oder synthetische direkte Thrombininhibitoren, wie z. B. Hirudin, PPACK (D-Phe-Pro-Arg-Chloromethylketon, HCl), Argatroban und Melagatran, oder natürliche oder synthetische direkte Faktor Xa-Inhibitoren, wie z. B. Antistasin, Tick Anticoagulant Peptide, Yagin, Draculin, GGACK (H-Glu-Glu-Arg-Chloromethylketon), Diamidino-Faktor Xa-Inhibitoren und Mono-Benzamidin Faktor Xa-Inhibitoren.

Das erfindungsgemäße Verfahren zur Bestimmung der Thrombozytenfunktion umfasst mehrere Verfahrensschritte. Zur Simulation der physiologischen Flussbedingungen, wie sie in kleinen Arterien herrschen, wird das Blut, das sich zunächst in einer Haltekammer befindet, durch eine Kapillare geleitet, die vorzugsweise einen Durchmesser von etwa 200 µm hat. Die Kapillare mündet in eine Messkammer, die von einem Trennelement in zwei Kompartimente unterteilt ist. Das Trennelement weist eine Öffnung auf, durch die das Blut vom ersten in das zweite Kompartiment durchgeleitet wird. Das Trennelement enthält mindestens einen Thrombozytenaktivator, wodurch das durch die Öffnung des Trennelements fließende Blut mit diesem mindestens einen im oder auf dem Trennelement enthaltenen Thrombozytenaktivator in Kontakt gebracht wird. Infolge der Thrombozytenaggregation, die durch den Kontakt mit dem mindestens einen Thrombozytenaktivator induziert wird, bildet sich ein Thrombozytenpfropf an der Öffnung des Trennelements. Es wird die Zeit gemessen, die für die Bildung des Thrombozytenpfropfs an der Öffnung des Trennelements bis zum Verschluss der Öffnung benötigt wird.

Bevorzugterweise wird die Verschlusszeit gemessen, indem ein Gerät verwendet wird, das einen Drucksensor enthält, welcher den Blutfluss durch die Apertur während des Tests bestimmt. Dabei wird nach anfänglicher, schneller Aspiration des Totvolumens der Messzelle zuerst der Initialflussrate ermittelt. Unterschreitet die Flussrate für mehr als 3 Sekunden den 10 % Wert dieser Initialflussrate, wird die Messung beendet und die bis dahin verstrichene Zeit als sogenannte Verschlusszeit angegeben. Diese sogenannte Verschlusszeit, die u. a. abhängig ist von der Aggregationsreaktion der stimulierten Thrombozyten, ist ein Maß für die Thrombozytenfunktion. Bevorzugterweise wird die Verschlusszeit, die für eine Vollblutprobe eines Patienten gemessen wurde, mit einem Verschlusszeitreferenzbereich für Vollblutproben gesunder Probanden verglichen.

Bevorzugterweise wird der Blutfluss durch die Kapillare und durch die Öffnung des Trennelements durch die Schaffung eines Unterdrucks in der Messkammer, also durch Ansaugen erzeugt. In einer besonders bevorzugten Auführungsform wird der Unterdruck durch das Zusammenwirken einer geeigneten Messzelle und einem Instrument erzeugt. Ein Beispiel für ein derartiges System ist z. B. in dem Patentdokument WO 97/034698 beschrieben.

Das erfindungsgemäße Verfahren zeichnet sich dadurch aus, dass die Vollblutprobe vor Durchleitung durch die Kapillare mit einem Aktivator von intrazellulären Adenylatzyklasen versetzt wird, und zwar entweder mit Prostaglandin E1 (PGE1) oder mit Forskolin. Prostaglandin E1 wird der Vollblutprobe in einer Endkonzentration von 11 nM bis 13 nM zugesetzt, Forskolin in wird der Vollblutprobe in einer Endkonzentration von 0.1 µM bis 5 µM zugesetzt.

Forskolin kann der Vollblutprobe in einer Endkonzentration von 0,5 µM bis 2,5 µM beziehungsweise von 1 µM bis 1,5 µM zugesetzt werden.

Die Vollblutprobe kann durch einfaches Vermischen mit einer Lösung, die mindestens einen Aktivator von intrazellulären Adenylatzyklasen enthält, mit dem gewünschten Aktivator versetzt werden. Dazu kann der mindestens eine Aktivator von intrazellulären Adenylatzyklasen z. B. entweder bereits im Entnahmemedium enthalten sein, welches zusätzlich ein Antikoagulanz enthält, oder die Lösung wird dem bereits antikoagulierten Vollblut zugegeben. Weiterhin ist es möglich, dass der mindestens eine Aktivator von intrazellulären Adenylatzyklasen in der Haltekammer der Vorrichtung enthalten ist, die für die Bestimmung der Verschlusszeit der Probe vorgesehen ist. In der Haltekammer kann der mindestens eine Aktivator von intrazellulären Adenylatzyklasen entweder in lyophilisierter oder in gelöster Form vorgelegt sein.

Das in dem erfindungsgemäßen Verfahren verwendete Trennelement enthält mindestens einen Thrombozytenaktivator zur Induktion der Thrombozytenaggregation. Das verwendete Trennelement kann z. B. einen Thrombozytenaktivator aus der Gruppe der Adenosin-Rezeptor-Aktivatoren enthalten, zu der insbesondere Adenosin-5'-diphosphat (ADP) und 2-Methylthioadenosin-5'-diphosphat (2-MeSADP) sowie deren Derivate gehören. In einer bevorzugten Ausführungsform wird ein Trennelement verwendet, das ein ADP-Salz oder ein 2-MeSADP-Salz enthält. In einer bevorzugten Ausführungsform wird ein Trennelement verwendet, das 1 bis 100 µg, besonders bevorzugt 10 bis 50 µg

ADP enthält. Ferner kann das verwendete Trennelement einen Thrombozytenaktivator aus der Gruppe Kollagen und Epinephrin enthalten, so dass 0,1 - 5 µg, besonders bevorzugt 1 µg Kollagen, oder 1 - 100 µg Epinephrin verwendet werden. In anderen bevorzugten Ausführungsformen werden Trennelemente verwendet, die ADP und Kollagen oder Epinephrin und Kollagen enthalten. Derartige Trennelemente sowie deren Herstellung und Verwendung sind z. B. in dem Patentdokument EP 716 744 B1 beschrieben.

In dem erfindungsgemäßen Verfahren kann ein Trennelement verwendet werden, das zusätzlich Calciumionen enthält, bevorzugterweise in Form von Calciumchlorid Dihydrat. Es kann ein Trennelement verwendet werden, das 50 bis 200 µg oder 100 bis 150 µg oder 125 µg Calciumionen in Form von Calciumchlorid Dihydrat enthält.

Das verwendete Trennelement ist eine poröse oder nicht-poröse Stützmatrix für den mindestens einen Thrombozytenaktivator zur Induktion der Thrombozytenaggregation und gegebenenfalls für Calciumionen. Bevorzugterweise ist das Trennelement in Form einer Membran ausgestaltet. Das bevorzugte Material ist flüssigkeitsabsorbierend, so dass die vorgenannten Substanzen in gelöster Form aufgebracht sein können. Besonders bevorzugte Materialien sind Celluloseester, Keramik, Nylon, Polypropylen, Polyethersulfon und Polyvinylidenfluorid (PVDF). Bevorzugterweise ist das mit den gewünschten Substanzen benetzte oder durchtränkte Trennelement getrocknet. Durch den Kontakt des Blutes mit dem Trennelement werden die Substanzen aus dem Trennelement gelöst und vermischen sich mit der Blutprobe.

Es wurde gefunden, dass wenn bei dem erfindungsgemäßen Verfahren ein Trennelement verwendet wird, das mindestens einen Thrombozytenaktivator aus der Gruppe der Adenosin-Rezeptor-Aktivatoren enthält, wie z. B. ADP (siehe auch Tabelle 2), die thrombozytenaggregationshemmende Wirkung der Acetylsalicylsäure (ASS) so stark minimiert werden kann, dass eine präzise Bestimmung der thrombozytenaggregationshemmenden (antithrombotischen) Wirkung anderer Thrombozytenaggregationshemmer, wie z. B. von P2Y(12)-Antagonisten wie Clopidogrel, auch in solchen Proben möglich ist, die ASS enthalten.

Das erfindungsgemäße Verfahren wird besonders bevorzugt zur Bestimmung der antithrombotischen (thrombozytenaggregationshemmenden) Wirkung eines P2Y(12)-Antagonisten verwendet, inbesondere zur Bestimmung eines eines P2Y(12)-Antagonisten aus der Gruppe: Clopidogrel, Ticlopidin, Prasugrel (synonym: CS-747) und andere Thienopyridine, AR-C67085MX (2-Propylthio-D-β,γ-dichlormethylen-Adenosin-5'-triphosphat), Cangrelor (synonym: AR-C69931MX, N⁶-[2-(methylthio)-ethyl]-2-(3,3,3-trifluoropropyl)thio-5'-Adenylsäure), C1330-7 (N¹-(6-Ethoxy-1,3-benzothiazol-2-yl-2-(7-ethoxy-4-hydroxy-2,2-dioxo-2H-2-⁶benzo[4,5][1,3]thiazolo[2,3-c][1,2,4]thiadiazin-3-yl)-2-oxo-1-ethansulfonamid), AZD 6140 (Nukleosidanalogon), MRS 2395 (2,2-Dimethyl-propionsäure 3-(2-chloro-6-methylaminopurin-9-yl)-2-(2,2-dimethyl-propionyloxymethyl)-propylester) und 2-MeSAMP (2-Methylthioadenosin-5'-monophospat).

Überraschenderweise wurde weiterhin gefunden, dass das erfindungsgemäße Verfahren ebenso zur Bestimmung der antithrombotischen (thrombozytenaggregationshemmenden) Wirkung eines P2Y(1)-Antagonisten verwendet werden kann. Insbesondere kann das Verfahren verwendet werden zur Bestimmung der antithrombotischen Wirkung von P2Y(1)-Antagonisten aus der Gruppe: MRS 2179 [2'-Deoxy-N⁶-methyladenosin 3',5'-diphosphat-Diammoniumsalz], MRS 2279 [(N)-Methanocarba-N⁶-methyl-2-chloro-2'-deoxyadenosin-3',5'-bisphosphat], MRS 2500 [2-lodo-N⁶-methyl-(N)-methanocarba-2'-deoxyadenosin-3',5'-bisphosphat], A2P5P [Adenosin-2',5'-bisphosphat], A3P5P [Adenosin-3',5'-bisphosphat] und A3P5PS [Adenosin-3'-phosphat,5'-phosphosulfat].

Das Trennelement weist eine bevorzugt kreisförmige Öffnung auf, die der Stützmatrix vorzugsweise durch Ausstanzen beigebracht wurde. Der Durchmesser der Öffnung im Trennelement ist derart bemessen, dass unter den Bedingungen des jeweiligen Verfahrens ein Thrombozytenpfropf gebildet werden kann, der die Öffnung verschließen und dadurch den Blutfluss stoppen kann. Vorzugsweise weist die Öffnung im Trennelement einen Durchmesser zwischen ungefähr 100 µm und ungefähr 200 µm auf. Besonders bevorzugt beträgt der Durchmesser der Öffnung im Trennelement etwa 150 µm.

Die folgenden Ausführungsbeispiele dienen der Veranschaulichung des erfindungsgemäßen Verfahrens und sind nicht als Einschränkung zu verstehen.

### Figuren

### Figur 1

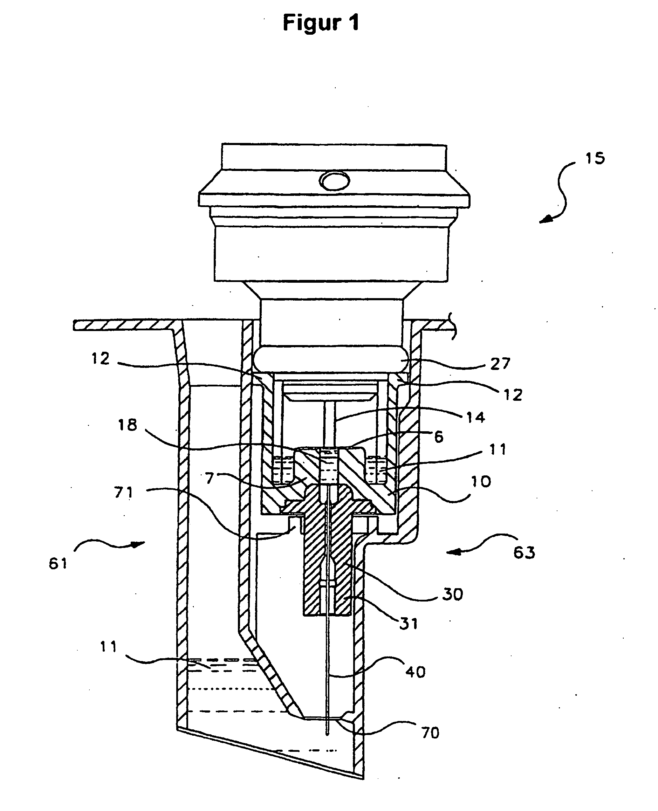

Figur 1 zeigt beispielhaft, wie eine Vorrichtung, die sich zur Durchführung des erfindungsgemäßen Verfahrens zur Bestimmung der Thrombozytenfunktion eignet, ausgestaltet sein kann. Gezeigt ist eine Messzelle gemäß WO 97/34698 im Längsschnitt, die zur Durchführung des erfindungsgemäßen Verfahrens in einem passenden Instrument plaziert ist und in welche ein Vakuumgerät (15) ragt, das für die Erzeugung des Unterdrucks verantwortlich ist. Das Vakuumgerät (15) weist dazu eine Ringdichtung (27) auf, die dichtend auf dem umlaufenden Rand (12) des Probenbechers (10) liegt. Die Messzelle weist ein Gehäuse auf, das eine Haltekammer (61) und eine Testkammer (63) bildet. Die Testkammer (63) ist zur Aufnahme eines Probenbechers (10) ausgebildet, dessen Lumen auch als Messkammer bezeichnet werden kann. Der Probenbecher (10) stützt ein mit Reagenzien behandeltes Trennelement (6) mit einer zentralen Öffnung (Apertur) und einen Kapillaransatz (30, 31), der eine Verbindung der Kapillare (40) mit dem Probenbecher (10) herstellt. Haltekammer (61) und Testkammer (63) sind durch ein durchdringbares Element (70) getrennt. Die Figur zeigt eine Phase aus dem Testzyklus, nachdem das Vakuumgerät (15) den Probenbecher (10) berührt und nach unten bewegt hat, so dass der Boden des Probenbechers (10) das Stützelement (71) berührt hat und die Kapillare (40) das durchdringbare Element (70) durchdrungen hat und in die Probe (11) eingedrungen ist. Das Instrument erzeugt nun einen Unterdruck im Probenbecher (10), wodurch die Probe (11) durch die Kapillare (40) in das erste Kompartiment (18) der Messkammer und anschließend durch die Öffnung im Trennelement (6) nach oben gesogen wird.

### Figur 2

Diagramm zur Darstellung der Verschlusszeiten (in Sekunden [s]), die für normale, unbehandelte Vollblutproben (Kontrolle) und für Vollblutproben, die mit dem P2Y(12)-Antagonisten MRS 2395 oder dem COX-1-lnhibitor Acetylsalicylsäure (ASS) in vitro versetzt wurden (siehe Beispiel 1). Es wurden mit gepuffertem Natriumcitrat antikoagulierte Vollblutproben von 11 gesunden Spendern verwendet. Es sind die Mittelwerte und die Standardabweichungen der Verschlusszeiten dargestellt, die mit Kol/Epi-Messzellen ermittelt wurden (Cut-off: 158 Sekunden). Im linken Block sind die Verschlusszeiten von Proben dargestellt, die gemäß dem Stand der Technik nicht mit einem Aktivator von intrazellulären Adenylatzyklasen versetzt wurden (Unbehandelt). Im mittleren und rechten Block sind die Verschlusszeiten von Proben dargestellt, die erfindungsgemäß vor Durchleitung durch die Kapillare mit PGE1 bzw. mit Forskolin versetzt wurden.

### Figur 3

Diagramm zur Darstellung der Verschlusszeiten (in Sekunden [s]), die für normale, unbehandelte Vollblutproben (Kontrolle) und für Vollblutproben, die mit dem P2Y(12)-Antagonisten MRS 2395 oder dem COX-1-lnhibitor Acetylsalicylsäure (ASS) in vitro versetzt wurden (siehe Beispiel 1). Es wurden mit gepuffertem Natriumcitrat antikoagulierte Vollblutproben von 11 gesunden Spendern verwendet. Es sind die Mittelwerte und die Standardabweichungen der Verschlusszeiten dargestellt, die mit Kol/ADP-Messzellen ermittelt wurden (Cut-off: 115 Sekunden). Im linken Block sind die Verschlusszeiten von Proben dargestellt, die gemäß dem Stand der Technik nicht mit einem Aktivator von intrazellulären Adenylatzyklasen versetzt wurden (Unbehandelt). Im mittleren und rechten Block sind die Verschlusszeiten von Proben dargestellt, die erfindungsgemäß vor Durchleitung durch die Kapillare mit PGE1 bzw. mit Forskolin versetzt wurden.

### Beispiele

### Beispiel 1: Verwendung des erfindungsgemäßen Verfahrens zur Bestimmung der antithrombotischen Wirkung eines P2Y(12)-Antagonisten und der Acetylsalicylsäure in vitro mit den Standard-Messzellen Kol/Epi und Kol/ADP

### 1a) Probenvorbereitung

11 gesunden Spendern wurde venöses Blut entnommen und mit Natriumcitrat antikoaguliert (3,2 % gepuffertes Na-Citrat).

Aliquots der Citratvollblutproben wurden in vitro mit dem P2Y(12)-Antagonisten MRS 2395 (Sigma-Aldrich Chemie GmbH, Steinheim, Deutschland) versetzt. Dazu wurde eine ethanolische MRS 2395-Stammlösung (15 mg/mL) mit den Vollblutproben vermischt, so dass eine Endkonzentration von 100 µmol/L erreicht wurde.

Weitere Aliquots der Citratvollblutproben wurden in vitro mit dem COX-1-lnhibitor Acetylsalicylsäure (kurz: ASS; Sigma-Aldrich Chemie GmbH, Steinheim, Deutschland) versetzt. Dazu wurde eine wässrige ASS-Stammlösung (1 mg/mL) mit den Vollblutproben vermischt, so dass eine Endkonzentration von 30 µmol/L erreicht wurde.

Nach Zugabe der Reagenzien wurden die Blutproben für 5 Minuten bei Raumtemperatur inkubiert.

### 1b) Bestimmung der antithrombotischen Wirkung von MRS 2395 mittels ADP-induzierter Lichttransmissionaggregometrie (nach Born)

Um zu überprüfen, ob die MRS 2395-behandelten Proben tatsächlich eine verminderte Thrombozytenaggregation zeigen, wurde aus Aliquots der unter Beispiel 1a) beschriebenen unbehandelten und MRS 2395-behandelten Vollblutproben plättchenreiches (PRP) und plättchenarmes Plasma (PPP) hergestellt, und die Proben wurden anschließend mit 5 µM ADP versetzt. Die PPP-Proben wurden als Leerwertkontrollen verwendet. Die photometrische Messung der Aggregationsreaktion wurde in dem automatischen Gerinnungsgerät BCT^{®} (Dade Behring Marburg GmbH, Marburg, Deutschland) unter kontinuierlichem Rühren (600 U/min) vorgenommen. Die Thrombozytenaggregation der MRS 2395-behandelten Proben war gegenüber der Thrombozytenaggregation der unbehandelten Proben im Mittel um 27 % vermindert.

### 1c) Ermittlung der Referenzbereiche für Kol/Epi- und Kol/ADP-Messzellen

Gesunden Spendern wurde venöses Blut entnommen und mit Natriumcitrat antikoaguliert (3,2 % gepuffertes Na-Citrat). Für jede Vollblutprobe wurde die Verschlusszeitbestimmung im PFA-100^{®}-Gerät durchgeführt. Mit einer Kol/Epi PFA-100^{®}-Messzelle [siehe Beispiel 1d)] und einer Kol/ADP PFA-100^{®}-Messzelle [siehe Beispiel 1d)] wurden Proben von 186 Spendern in Doppelbestimmung untersucht.

Der Referenzbereich (Normalbereich) für die Kol/Epi-Verschlusszeit bzw. für die Kol/ADP-Verschlusszeit wurde festgelegt, indem der Messwertebereich bestimmt wurde, in dem 90 % der Messwerte lagen, die für die gesunden Probanden bestimmt worden waren (90 % Zentralinterval der Normalverteilung aller Messungen). Damit ergaben sich folgende Referenzbereiche für die Verschlusszeiten:

| | | |
|---|---|---|
| Kol/Epi | 70 - 158 | Sekunden |
| Kol/ADP | 60 - 115 | Sekunden. |

Als Cut-Off, d. h. als Grenzwert für eine Thrombozytendysfunktion wurde die obere Referenzgrenze des Referenzbereichs festgelegt. Weicht die Verschlusszeit einer Patientenprobe vom Referenzbereich ab, kann dies für eine Störung der Thrombozytenfunktion sprechen. Das heisst, Kol/Epi-Verschlusszeiten, die größer als 158 Sekunden sind bzw. Kol/ADP-Verschlusszeiten, die größer als 115 Sekunden sind, weisen auf das Vorliegen einer Thrombozytendysfunktion im Sinne einer verminderten Aggregationsfähigkeit hin.

### 1d) Bestimmung der antithrombotischen Wirkung von MRS 2395 und Acetylsalicylsäure mit Hilfe des erfindungsgemäßen Verfahrens unter Flussbedingungen

Zur Bestimmung der Verschlusszeit mit Hilfe einer Kol/Epi PFA-100^{®}-Messzelle (2 µg Kollagen und 10 µg Epinephrin auf dem Trennelement; 150 µm Aperturdurchmesser; Dade Behring Marburg GmbH, Marburg, Deutschland) bzw. mit Hilfe einer Kol/ADP PFA-100^{®}-Messzelle (2 µg Kollagen und 50 µg ADP auf dem Trennelement; 150 µm Aperturdurchmesser; Dade Behring Marburg GmbH, Marburg, Deutschland) als Maß für die Thrombozytenfunktion wurden die unter Beispiel 1a) beschriebenen Vollblutproben in einem PFA-100^{®} Gerät (Platelet Function Analyzer-100, Dade Behring Marburg GmbH, Marburg, Deutschland) untersucht.

Es wurden Aliquots der unter 1a) beschriebenen Proben entnommen und erfindungsgemäß entweder mit Prostaglandin E1 (kurz: PGE1) oder mit Forskolin (beide bezogen durch Sigma-Aldrich Chemie GmbH, Steinheim, Deutschland) zur Aktivierung von intrazellulären Adenylatzyklasen versetzt. Dazu wurden die Proben mit einer ethanolischen PGE1-Stammlösung (0,05 mg/mL) bzw. einer ethanolischen Forskolin-Stammlösung (5 mg/mL) vermischt, so dass eine PGE1-Endkonzentration von 12,5 nM bzw. eine Forskolin-Endkonzentration von 1 µM erreicht wurde. Weitere Aliquots der unter 1a) beschriebenen Proben wurden nicht mit einem Aktivator von intrazellulären Adenylatzyklasen versetzt und dienten als Kontrolle.

Es wurden dann jeweils 800 µL der mit PGE1 oder Forskolin versetzten Blutproben bzw. der Kontrollproben in die Haltekammer einer Kol/Epi- oder Kol/ADP-Messzelle (+37 °C) gegeben und für 3 Minuten bei +37 °C im Gerät inkubiert. Anschließend wurde durch das Gerät ein Unterdruck von -40 mbar erzeugt, wodurch das Blut aus der Haltekammer durch eine Kapillare (Durchmesser 200 µm) und schließlich durch eine Öffnung (Apertur) des Trennelements in der Messkammer gesaugt wurde. Als Verschlusszeit wurde die Zeit bestimmt, die bis zum Verschluss der Apertur durch Bildung eines Blutgerinnsels benötigt wurde. Jede der untersuchten Proben wurde doppelt bestimmt, und als Messwert wurde der Mittelwert einer Doppelbestimmung verwendet.

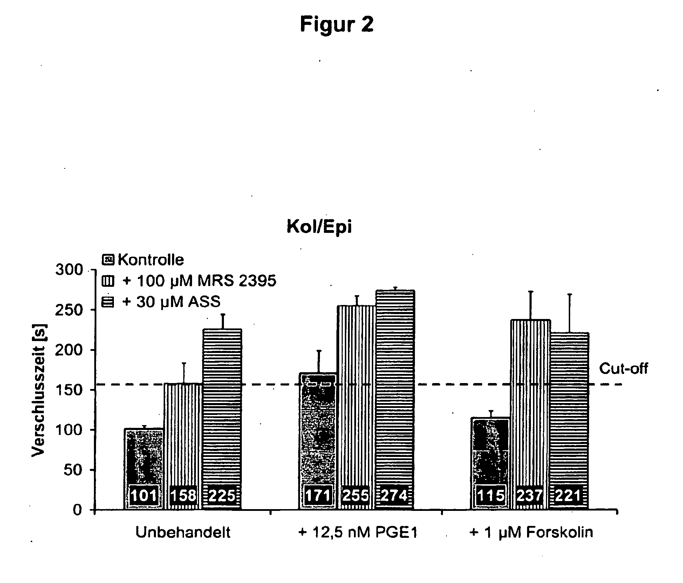

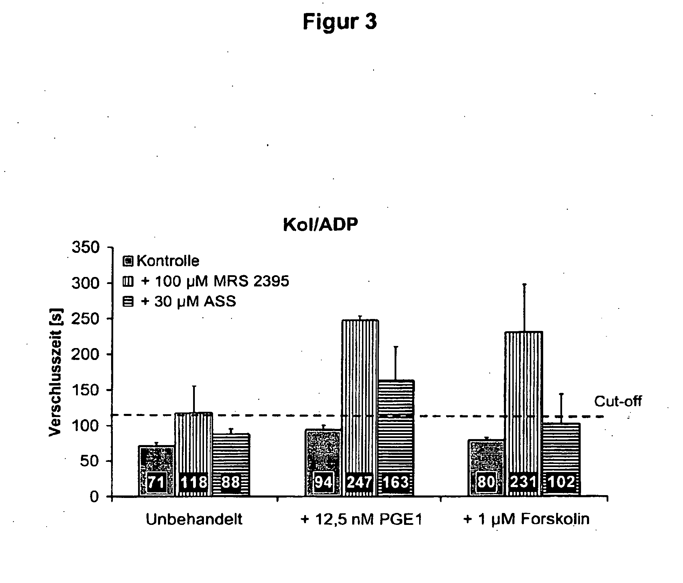

Die Ergebnisse der Untersuchungen sind in Figur 2 für die Kol/Epi-Messzelle und in Figur 3 für die Kol/ADP-Messzelle in Verbindung mit den dazugehörigen Figurenbeschreibungen zusammengefasst.

In Tabelle 1 und Tabelle 2 ist im Einzelnen aufgeführt, für wieviele der jeweils 11 MRS 2395- oder Acetylsalicylsäure-behandelten Proben mit Hilfe des erfindungsgemäßen Verfahrens unter Verwendung einer konventionellen Kol/Epi-Messzelle (Tabelle 1) bzw. Kol/ADP-Messzelle (Tabelle 2) eine Verschlusszeit oberhalb des Cut-Offs gemessen wurde.

### Kol/Epi-Messzelle

Mit der Kol/Epi-Messzelle wird ohne Zugabe eines Aktivators von intrazellulären Adenylatzyklasen (Kontrolle) in nur 3 von 11 MRS 2395-behandelten Proben und in 8 von 11 ASS-behandelten Proben eine abnormal verminderte Thrombozytenaggregation gemessen. Durch Anwendung des erfindungsgemäßen Verfahrens, d. h. durch vorheriges Vermischen der Proben mit PGE1 oder Forskolin, werden bei Verwendung von PGE1 in allen MRS 2395- und allen ASS-behandelten Proben und bei Verwendung von Forskolin in 9 von 11 MRS 2395-behandelten Proben und in allen ASS-behandelten Proben eine abnormal verminderte Thrombozytenaggregation nachgewiesen. Allerdings führt die verwendete Prostaglandin E1-Menge auch zu einer signifikanten Verlängerung der Verschlusszeiten der Kontrollen, während bei der verwendeten Forskolinmenge nur eine geringfügige Verlängerung der Verschlusszeiten der Kontrollen zu beobachten ist. Die Anwendung des erfindungsgemäßen Verfahrens führt zu einer deutlichen Erhöhung der Sensitivität der Kol/Epi-Messzelle für eine durch die Blockierung des P2Y(12)-Rezeptors oder durch Inhibierung der Cyclooxygenase-1 verursachte Thrombozytendysfunktion.

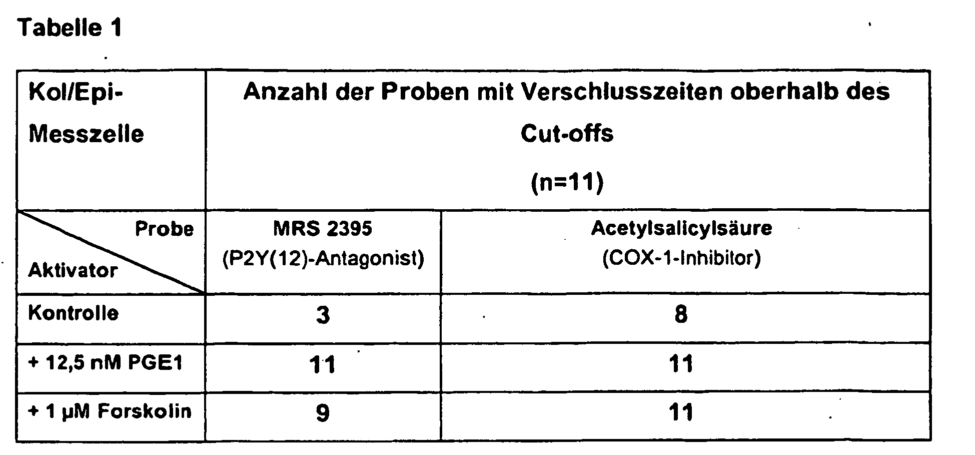

### Kol/ADP-Messzelle

Mit der Kol/ADP-Messzelle wird ohne Zugabe eines Aktivators von intrazellulären Adenylatzyklasen (Kontrolle) in nur 2 von 11 MRS 2395-behandelten Proben und in nur 1 von 11 ASS-behandelten Proben eine abnormal verminderte Thrombozytenaggregation gemessen. Durch Anwendung des erfindungsgemäßen Verfahrens, d. h. durch vorheriges Vermischen der Proben mit PGE1 oder Forskolin, werden bei Verwendung von PGE1 in allen MRS 2395-behandelten Proben und in 4 von 11 ASS-behandelten Proben und bei Verwendung von Forskolin in 10 von 11 MRS 2395-behandelten Proben und in keiner der ASS-behandelten Proben eine abnormal verminderte Thrombozytenaggregation nachgewiesen. Weder die verwendete Forskolinmenge noch die verwendete Prostaglandin E1-Menge führten zu einer signifikanten Verlängerung der Verschlusszeiten der Kontrollen. Durch die Zugabe von 1 µM Forskolin kann die Sensitivität für die Blockierung des P2Y(12)-Rezeptors deutlich erhöht werden, ohne dadurch eine signifikante Sensitivität für die ASS-induzierte Thrombozytendysfunktion zu verursachen, wie es bei der Zugabe von 12,5 nM Prostaglandin der Fall ist.

Das erfindungsgemäße Verfahren eignet sich also aufgrund seiner hohen Sensitivität für P2Y(12)-Antagonisten-induzierte Thrombozytendysfunktionen und im Falle der Kol/ADP-Messzelle seiner geringen Sensitivität für Acetylsalicylsäure-induzierte Thrombozytendysfunktionen, zur Differenzierung der beiden Antithrombotika-Klassen.

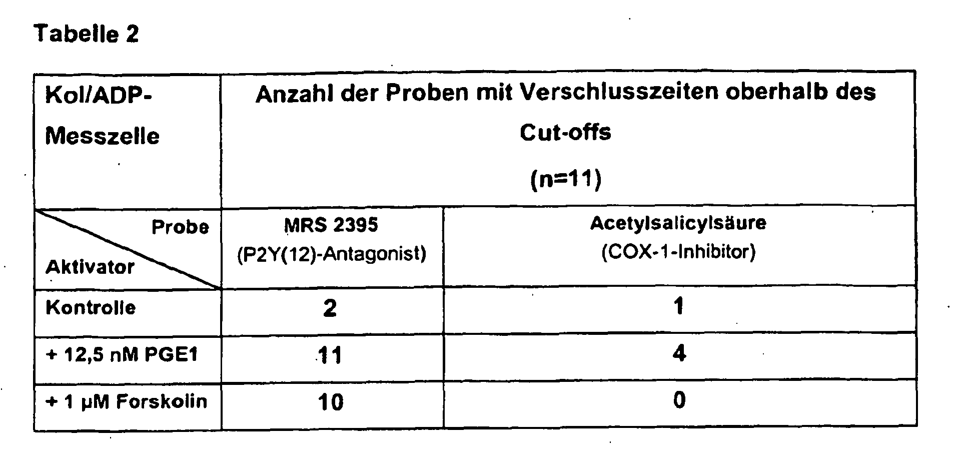

## Patentansprüche

1. Verfahren zur Bestimmung der Thrombozytenfunktion in einer Vollblutprobe, wobei das Verfahren folgende Schritte umfasst:
a) Durchleiten des Blutes durch eine Kapillare und anschließend durch eine Öffnung eines Trennelements, welches mindestens einen Thrombozytenaktivator aus der Gruppe der Adenosin-Rezeptor-Aktivatoren enthält; und
b) Messen der Zeit, die für die Bildung eines Thrombozytenpfropfs an der Öffnung des Trennelements bis zum Verschluss der Öffnung benötigt wird;
**dadurch gekennzeichnet, dass**
die Vollblutprobe vor Durchleitung durch die Kapillare mit Prostaglandin E1 in einer Endkonzentration von 11 bis 13 nM oder mit Forskolin in einer Endkonzentration von 0,1 bis 5 µM versetzt wird.

2. Verfahren gemäß Anspruch 1 **dadurch gekennzeichnet, dass** das in Schritt a) verwendete Trennelement mindestens einen Adenosin-Rezeptor-Aktivator aus der Gruppe denosin-5'-diphosphat, 2-Methylthloadenosin-5'-diphosphat und deren Derivate enthält.

3. Verfahren gemäß einem der Ansprüche 1 bis 2 **dadurch gekennzeichnet, dass** das in Schritt a) verwendete Trennelement zusätzlich Kollagen enthält.

4. Verfahren gemäß einem der Ansprüche 1 bis 3 **dadurch gekennzeichnet, dass** der Vollblutprobe Forskolin in einer Endkonzentration von 0,5 bis 2,5 µM, besonders bevorzugt von 1,0 bis 1,5 µM zugesetzt wird.

5. Verfahren gemäß einem der Ansprüche 1 bis 4 **dadurch gekennzeichnet, dass** die Vollblutprobe mit Citrat, einem direkten Thrombininhibitor oder einem einem direkten Faktor Xa-Inhibitor antikoaguliert ist.

6. Verwendung eines Verfahrens gemäß einem der Ansprüche 1 bis 5 zur Bestimmung der antithrombotischen Wirkung eines P2Y(12)-Antagonisten.

7. Verwendung gemäß Anspruch 6 zur Bestimmung der antithrombotischen Wirkung eines P2Y(12)-Antagonisten aus der Gruppe Clopidogrel, Ticlopidin, Prasugrel, MRS 2395, AR-C67085MX, Cangrelor, C1330-7 und 2-Methylthioadenosin-5'-monophosphat.

8. Verwendung eines Verfahrens gemäß einem der Ansprüche 1 bis 5 zur Bestimmung der antithrombotischen Wirkung eines P2Y(1)-Antagonisten.

9. Verwendung gemäß Anspruch 8 zur Bestimmung der antithrombotischen Wirkung eines P2Y(1)-Antagonisten aus der Gruppe MRS 2179, MRS 2279, MRS 2500, Adenosin-2',5'-bisphosphat, Adenosin-3',5'-bisphosphat und Adenosin-3'-Phosphat,5'-phosphosulfat.

## Claims

1. A method for the determination of platelet function in a whole blood sample wherein the method has the following steps:
a) passing the blood though a capillary and then through an opening of a divider which comprises at least one platelet activator from the group of adenosine receptor activators; and
b) measurement of the time that is required for the formation of a thrombus at the opening of the divider up to closure of the opening;
wherein
the whole blood sample is mixed with prostaglandin E1 in a final concentration of from 11 to 13 nM or with forskolin in a final concentration of from 0.1 to 5 µM before passing through the capillary.

2. The method as claimed in claim 1, wherein the divider used in step a) comprises at least one adenosine receptor activator from the group adenosine 5'-diphosphate, 2-methylthioadenosine 5'-diphosphate and their derivatives.

3. The method as claimed in either of claims 1 to 2, wherein the divider used in step a) additionally comprises collagen.

4. The method as claimed in any of claims 1 to 3, wherein forskolin is added in a final concentration of from 0.5 to 2.5 µM, particularly preferably from 1.0 to 1.5 µM, to the whole blood sample.

5. The method as claimed in any of claims 1 to 4, wherein the whole blood sample is anticoagulated with citrate, a direct thrombin inhibitor or a direct factor Xa inhibitor.

6. The use of a method as claimed in any of claims 1 to 5 for the determination of the antithrombotic action of a P2Y(12) antagonist.

7. The use as claimed in claim 6 for the determination of the antithrombotic action of a P2Y(12) antagonist from the group clopidogrel, ticlopidine, prasugrel, MRS 2395, AR-C67085MX, cangrelor, C1330-7, and 2-methylthioadenosine 5'-monophosphate.

8. The use of a method as claimed in any of claims 1 to 5 for the determination of the antithrombotic action of a P2Y(1) antagonist.

9. The use as claimed in claim 8 for the determination of the antithrombotic action of a P2Y(1) antagonist from the group MRS 2179, MRS 2279, MRS 2500, adenosine 2',5'-bisphosphate, adenosine 3',5'-bisphosphate and adenosine 3'-phosphate 5'-phosphosulfate.

## Revendications

1. Procédé de détermination de la fonction des thrombocytes dans un échantillon de sang complet, le procédé comprenant les stades suivants :
a ) on fait passer le sang dans un capillaire et ensuite dans une ouverture d'un élément de séparation, qui contient au moins un activateur de thrombocytes choisi dans le groupe des activateurs de récepteur d'adénosine ; et
b ) on mesure le temps qui est nécessaire pour la formation d'une goutte de thrombocytes sur l'ouverture de l'élément de séparation jusqu'à la fermeture de l'ouverture ;
**caractérisé en ce que**
l'on mélange l'échantillon de sang complet, avant de faire passer dans le capillaire, à de la prostaglandine E1 en une concentration finale de 11 à 13nM ou à de la forskoline en une concentration finale de 0,1 à 5µM.

2. Procédé suivant la revendication 1, **caractérisé en ce que** l'élément de séparation utilisé au stade a ) contient au moins un activateur de récepteur d'adénosine choisi dans le groupe du 5'-diphosphate d'adénosine, du 5'-diphosphate de 2 méthylthioadénosine et de leurs dérivés.

3. Procédé suivant l'une des revendications 1 à 2, **caractérisé en ce que** l'élément de séparation utilisé au stade a ) comprend, en outre, du collagène.

4. Procédé suivant l'une des revendications 1 à 3, **caractérisé en ce qu'**on ajoute un échantillon de sang complet de la forskoline en une concentration finale de 0,5 à 2,5µM, en particulier de manière préférée de 1,0 à 1,5 µM.

5. Procédé suivant l'une des revendications 1 à 4, **caractérisé en ce que** l'on anticoagule l'échantillon de sang complet par du citrate, par un inhibiteur directe de thrombine ou par un inhibiteur direct du facteur Xa.

6. Utilisation d'un procédé suivant l'une des revendications 1 à 5, pour la détermination de l'effet anti-thrombotique d'un antagoniste de P2Y( 12 ).

7. Utilisation suivant la revendication 6 de détermination d'effet anti-thrombotique d'un antagoniste de P2Y( 12 ) choisi dans le groupe Clopidogrel, Ticlopidine, Prasugrel, MRS 2395, AR-C67085MX, Cangrelor, C1330-7 et 5'-monophosphate de 2-méthylthioadénosine.

8. Utilisation d'un procédé suivant l'une des revendications 1 à 5, pour la détermination de l'effet anti-thrombotique d'un antagoniste de P2Y( 1 ).

9. Utilisation suivant la revendication 8, pour la détermination de l'effet anti-thrombotique d'un antagoniste de P2Y( 1 ) du groupe MRS 2179, MRS 2279, MRS 2500, 2'5'-disphosphate d'adénosine, 3'5'-bisphosphate d'adénosine et 3'-phosphate, 5'-phosphosulfate d'adénosine.
